**Europäisches Patentamt**

**European Patent Office**

**Office européen des brevets**

(11) Veröffentlichungsnummer: **0 119 454**
**A2**

# (12) EUROPÄISCHE PATENTANMELDUNG

(21) Anmeldenummer: **84101438.4**

(51) Int. Cl.³: **A 61 K 7/06**

(22) Anmeldetag: **13.02.84**

(30) Priorität: **21.02.83 DE 3305964**

(43) Veröffentlichungstag der Anmeldung: **26.09.84**
**Patentblatt 84/39**

(84) Benannte Vertragsstaaten: **AT BE CH DE FR GB IT LI LU NL SE**

(71) Anmelder: **Henkel Kommanditgesellschaft auf Aktien,**
**Postfach 1100 Henkelstrasse 67,**
**D-4000 Düsseldorf-Holthausen (DE)**

(72) Erfinder: **Ritter, Wolfgang, Dr., Hochdahler Strasse 32,**
**D-4010 Hilden (DE)**
Erfinder: **Tenhaef, Rolf, Vennstrasse 41,**
**D-4000 Düsseldorf 12 (DE)**
Erfinder: **Höffkes, Horst, Dr., Carlo-Schmidt-Strasse 113,**
**D-4000 Düsseldorf (DE)**
Erfinder: **Seidel, Kurt, Nosthoffenstrasse 59,**
**D-4000 Düsseldorf 13 (DE)**

(54) **Verwendung von kationischen Polymeren als antistatische Zusätze zu Haarbehandlungsmitteln.**

(57) Die Verwendung kationischer Polymerer, die erhältlich sind durch Epoxidation von 1,3-Dien-Homo- oder Copolymerisaten, Umsetzung der Epoxide mit niedermolekularen, primären, sekundären oder tertiären Aminen und Überführung in die Salzform oder in polymere quartäre Ammoniumsalze in haarkosmetischen Mitteln verleiht diesen antistatische Wirkungen. Bevorzugt zu verwendende kationische Polymere werden durch Epoxidation von Polybutadien, welches zu über 50% 1,4-Cis-Konfiguration aufweist, Umsetzung mit Dimethylamin oder Morpholin und Überführung in die Hydrochloride oder Alkylierung mit Glycidyltrimethylammoniumchlorid, 3-Chlor-2-hydroxypropyltrimethylammoniumchlorid oder Ethylenoxid hergestellt. Bevorzugt erfolgt die Verwendung in Shampoos in Mengen von 0,5–5,0 Gew.-% neben 5–30 Gew.-% Alkylethersulfattensid und 1–5 Gew.-% eines zwitterionischen oder amphoteren Waschaktivstoffes.

Henkelstraße 67

4ooo Düsseldorf, den 22. 9. 1983

- 1 -

0119454

HENKEL KGaA

ZR-FE/Patente

Dr.JG/Po

P a t e n t a n m e l d u n g

<u>D 6699</u> EP

**"Verwendung von kationischen Polymeren als antistatische Zusätze zu Haarbehandlungsmitteln"**

Das Haupthaar weist nach dem Waschen mit Shampoos, Dusch- und Badepräparaten auf Basis von synthetischen Tensiden, vor allem aber nach kosmetischen Behandlungen wie Färben und Verformen oft einen kosmetisch unbefriedigenden Zustand auf. Es ist im nassen Zustand nur schwer zu kämmen und weist im trockenen Zustand wenig Halt und Fülle auf. Vor allem neigt es zur statischen Aufladung, wodurch das "Fliegen" des frisch gewaschenen Haares verursacht wird.

Es ist bekannt, nach dem Waschen, Färben oder Dauerwellen des Haares konditionierende Präparate auf das Haar einwirken zu lassen. Solche Präparate enthalten als Wirkstoffe meist kationische grenzflächenaktive Stoffe. Es ist auch bekannt, üblichen Shampoos auf Basis anionaktiver Tenside oder auf Basis von Mischungen von Tensiden unterschiedlicher Ionogenität bestimmte Substanzen beizufügen, um gleichzeitig mit der Haarwäsche einen gewissen konditionierenden Effekt zu erzielen. Solche Substanzen sind z.B. wasserlösliche Proteine oder Proteinabbauprodukte, polykationische Polymere wie z.B. die in der DE-OS 21 09 081 beschriebenen quartäre Ammoniumgruppen aufweisenden synthetischen Polymeren oder die aus der DE-OS 24 23 833 bekannten, quartären Stickstoff enthaltenden Celluloseether.

...

0119454
HENKEL KGaA
ZR-FE/Patente

- 2 -

Die bekannten konditionierenden Wirkstoffe weisen aber erhebliche Mängel auf. Die kationischen Tenside sind zwar aus nichtionogenen Formulierungen gut wirksam, in Shampoos oder z.B. Haarfärbemitteln mit anionischen Tensiden lassen sich die kationischen Tenside aber wegen der begrenzten Verträglichkeit mit Aniontensiden nicht oder nur in praktisch unwirksamen Mengen einsetzen. Die Aniontensid-Komplexe mit quartären Ammoniumverbindungen führen darüber hinaus zu starker "Belastung" des Haares, was sich in einem fettigen Aussehen und geringer Fülle des behandelten Haares zeigt.

Diese Haarbelastung ist auch in mehr oder weniger starkem Umfang bei den bekannten Formulierungen zur Haarnachbehandlung auf kationaktiver Basis zu verzeichnen.

Die polykationischen Polymeren bewirken zwar meist eine befriedigende Verbesserung der Naßkämmbarkeit, die statische Aufladbarkeit des trockenen Haares wird jedoch durch diese Zusätze kaum verringert. In vielen Fällen, insbesondere in anionischen oder anionisch-amphoteren Shampooformulierungen, können kationische Polymere sogar zu einer Verstärkung der statischen Aufladbarkeit des trockenen Haares führen.

Es bestand daher die Aufgabe, konditionierende Wirkstoffe aufzufinden, welche als konditionierende und antistatisch wirksame Zusätze zu Haarbehandlungsmitteln geeignet sind und die geschilderten Nachteile nicht oder in erheblich geringerem Maße aufweisen.

Gegenstand der Erfindung ist die Verwendung von kationischen Polymeren, die man erhalten kann durch

...

- Epoxidation von 1.3-Dien-Homo-     oder -copolymerisaten aus mindestens 10 1,3-Dien-Einheiten bis
zu einem Umsatz von mindestens 10 % der vorhandenen
Doppelbindungen,

- Umsetzung der epoxidierten Poly-1,3-diene mit primären,
sekundären oder tertiären Aminen der allgemeinen Formel
$R^1NR^2R^3$, in der $R^1$, $R^2$ und $R^3$ Wasserstoff, eine
Niedrigalkylgruppe mit 1 - 4 C-Atomen, eine Hydroxyalkylgruppe mit 2 - 4 C-Atomen oder $R^2$ und $R^3$ gemeinsam
mit dem Stickstoffatom einen Morpholin-, Piperidin- oder
Piperazinring bilden und wenigstens eine Gruppe $R^1$, $R^2$
oder $R^3$ ungleich Wasserstoff ist,

- Überführung der Umsetzungsprodukte mit Mineralsäuren oder niedermolekularen Carbonsäuren in die Salzform, bevorzugt in die Hydrochloride, oder Überführung der polymeren tertiären Amine in quartäre
Ammoniumsalze durch Alkylierung mit Verbindungen aus
der Gruppe 3-Chlor-2-hydroxypropyltrimethylammonium-
chlorid, RCl, RBr, $R_2SO_4$, worin R eine Methyl- oder
Ethylgruppe darstellt, oder durch Anlagerung von
Ethylenoxid, Propylenoxid, Glycid oder Glycidyltrimethylammoniumchlorid und Überführung in die Salzform, bevorzugt in die Hydrochloride,

als antistatisch wirksame Zusätze in haarkosmetischen
Mitteln.

Die für die erfindungsgemäße Verwendung geeigneten kationischen Polymeren bzw. die Verfahren zu ihrer Herstellung sind bekannt:

...

Die Epoxidation von Butadienoligomeren ist z.B. in der Chemiker-Zeitung 95 (1971) Nr. 20, S. 857 - 863 beschrieben. Die Umsetzung epoxidierter Polybutadiene mit tertiären Aminen unter Bildung von quartären Ammoniumpolymeren ist aus DE-OS 21 41 941 bekannt. Die Umsetzung epoxidierter Butadienhomo- oder -copolymerisate mit primären und sekundären aliphatischen und cycloaliphatischen Aminen ist in DE-OS 27 32 736 beschrieben. Die Überführung der polymeren tertiären Amine, die bei der Umsetzung der Polyepoxide mit sekundären Aminen entstehen, in polymere quartäre Ammoniumsalze kann nach ebenfalls bekannten Verfahren durch Alkylierung mit 3-Chlor-2-hydroxypropyl-trimethylammoniumchlorid, Methylchlorid, Ethylchlorid, Dimethylsulfat, Diethylsulfat oder durch Anlagerung von 1,2-Monoepoxiden wie Ethylenoxid, Propylenoxid, Glycid oder Glycidyltrimethylammoniumchlorid erfolgen.

Als Ausgangsprodukte für die Herstellung der erfindungsgemäß zu verwendenden kationischen Polymeren eignen sich 1.3-Dien-Homo- oder -copolymerisate. Als 1.3-Diene kommen vor allem Butadien, 1.3-Pentadien, Isopren und Chloropren in Frage. Bevorzugt werden flüssige Butadienhomo- oder -copolymerisate eingesetzt. Als Comonomere können aber auch Monoolefine wie Ethylen, Propylen, Styrol oder Acrylsäurederivate enthalten sein. Diese Poly-1.3-diene sollen aber mindestens 10 1.3-Dien-Einheiten pro Molekül enthalten. Bevorzugt sind flüssige Butadienhomopolymerisate geeignet. Die in solchen Poly-1.3-dienen enthaltenen Doppelbindungen können je nach den Polymerisationsbedingungen cis-Konfiguration oder trans-Konfiguration aufweisen oder vinylständig angeordnet sein.

...

0119454
HENKEL KGaA
ZR-FE/Patente

Bevorzugt werden Polymerisate eingesetzt, die an den Doppelbindungen zu über 50 % 1,4-cis-Konfiguration aufweisen.

Die Epoxidation der Poly-1,3-diene soll bis zu einem Umsatz von mindestens 10 % der vorhandenen Doppelbindungen erfolgen. Bevorzugt sind Polyepoxide mit einem Gehalt von 3 - 6 Gew.-% Epoxid-Sauerstoff geeignet.

Für die Umsetzung mit den Polyepoxiden geeignete Amine sind z.B. Methylamin, Ethylamin, Isopropylamin, Monoethanolamin, Dimethylamin, Diethylamin, Diethanolamin, Morpholin, Piperidin, Piperazin, Trimethylamin, Triethylamin, Triethanolamin, N.N.-Dimethylethanolamin. Bevorzugt sind die Umsetzungsprodukte mit Dimethylamin und Morpholin.

Die Umsetzung mit tertiären Aminen führt direkt zu polymeren quartären Ammoniumverbindungen, die durch Umsetzung mit Mineralsäuren oder niedermolekularen Carbonsäuren wie Essigsäure, Milchsäure, Glykolsäure, in die Salzform überführt werden. Bevorzugt ist die Überführung mit Salzsäure in die Hydrochloride.

Die Umsetzung mit primären und sekundären Aminen führt zu polymeren sekundären und tertiären Aminen, die nach Überführung in die Salzform als kationische Polymere für die erfindungsgemäße Verwendung geeignet sind.

Die durch Umsetzung mit sekundären Aminen erhaltenen polymeren tertiären Amine können aber auch durch Alkylierung in polymere quartäre Ammoniumverbindungen überführt werden. Bevorzugt werden Umsetzungsprodukte von epoxidierten Poly-1,3-dienen mit Dimethylamin durch Alkylierung mit Glycidyltrimethylammoniumchlorid oder 3-Chlor-2-hydroxypropyltrimethylammoniumchlorid in poly-

...

- 6 -

mere quartäre Ammoniumsalze überführt. Eine weitere für die erfindungsgemäße Verwendung bevorzugte Gruppe von kationischen Polymeren wird durch Umsetzung der Poly-1,3-dien-Epoxide mit Dimethylamin, Quaternierung der Umsetzungsprodukte durch Anlagerung von Ethylenoxid und Überführung in die Salzform erhalten.

Durch die erfindungsgemäße Verwendung der wie beschrieben erhältlichen kationischen Polymeren in haarkosmetischen Mitteln erhalten diese eine deutliche antistatische Wirkung bei der Anwendung am menschlichen Haar, ohne die üblicherweise bei kationaktiven Verbindungen auftretende vermehrte Haarbelastung. Aufgrund dieser Wirkung wird mit solchen haarkosmetischen Mitteln Sitz und Fülle des Haares merklich verbessert. Diese Wirkung ist überraschend, da mit bekannten kationischen Polymeren kaum antistatische Effekte, insbesondere bei der Anwendung aus Aniontenside enthaltenden Formulierungen, zu erzielen sind.
Die Prüfung auf antistatische Wirksamkeit kann z.B. auf die folgende Weise durchgeführt werden:

Haarsträhnen von ca. 2 g Gewicht und 20 cm Länge werden durch einmaliges Blondieren und einmalige Kaltwellbehandlung mit marktüblichen Präparaten (ohne die erfindungsgemäß zu verwendenden kationischen Polymeren) vorbehandelt, gewaschen, gespült und getrocknet. Auf die so vorbehandelten Haarsträhnen wird mit einer Pipette 0,5 ml einer Prüflösung, die z.B. 1 Gew.-% des kationischen Polymeren enthält, verteilt und einmassiert. Anstelle der Prüflösung kann ein beliebiges haarkosmetisches Mittel mit einem Gehalt an den erfindungsgemäß zu verwendenden kationischen Polymeren ein-

. . .

gesetzt werden. Nach der Behandlung wird die Haarsträhne mit klarem, warmem (35°C) Wasser gespült, 30 Minuten bei 60°C getrocknet und 24 Stunden bei 20°C und 50 % rel. Luftfeuchtigkeit konditioniert.

Danach wird die Haarsträhne 20 mal mit einem Hartgummikamm durchgekämmt. Bei den unbehandelten Haarproben tritt dabei eine erhebliche statische Aufladung der Haarsträhne ein, die sich in einer deutlichen Spreizung der Haarspitzen bemerkbar macht.

Die mit den erfindungsgemäß zu verwendenden kationischen Polymeren oder diese enthaltenden Mitteln behandelten Haarsträhnen zeigen keine statische Aufladung, die Haare bleiben nach dem Kämmen parallel ausgerichtet.

Für die erfindungsgemäße Verwendung geeignete haarkosmetische Mittel sind z.B. Shampoos, Haarnachbehandlungsmittel, z.B. Haarspülungen, Haarkurpräparate, Haarwässer, Haarfestiger, Haarsprays, Haarfärbemittel und Haarwellmittel. Die Verwendung der Produkte ist aber auch in Badezusätzen, Duschbädern und flüssigen Körperwaschmitteln von Vorteil, da diese auch zur Reinigung der Haare verwendet werden oder bei der Anwendung auf das Haar gelangen.

Die erfindungsgemäß zu verwendenden kationischen Polymeren werden in die genannten haarkosmetischen Zubereitungen in Mengen von 0,1 - 10 Gew.-%, bevorzugt 0,5 - 5,0 Gew.-%, bezogen auf die gesamte Zubereitung, bei Aerosolpräparaten, bezogen auf die treibmittelfreie Wirkstofflösung, eingesetzt.

...

- 8 -

Besonders wirksam sind die erfindungsgemäß zu verwendenden kationischen Polymeren in haarkosmetischen Mitteln
mit einem Gehalt an anionischen oberflächenaktiven Stoffen, da aus solchen Zubereitungen ein antistatischer
Effekt sonst kaum zu erzielen ist. Geeignete anionische
oberflächenaktive Stoffe sind z.B.Alkali-, Magnesium-,
Ammonium- und/oder Alkanolammoniumsalze von Alkylschwefelsäurehalbestern mit 8 - 18, bevorzugt 12 - 16 C-Atomen
im Alkylrest (sog. Alkylsulfate) oder von Alkylpolyglykolether-schwefelsäurehalbestern mit 8 - 18, bevorzugt
12 - 16 C-Atomen im Alkylrest und 1 - 6 Glykolethergruppen im Molekül (sog. Alkylethersulfate). Weitere geeignete anionische oberflächenaktive Stoffe sind primäre
und sekundäre, lineare Alkansulfonate mit 10 - 18 C-Atomen, Alkensulfonate und Hydroxyalkansulfonate, wie sie
durch Sulfonierung von linearen Olefinen mit 10 - 18 C-
Atomen erhalten werden, Fettsäurealkylolamid- und Fettsäurealkylolamidpolyglykolethersulfate, sulfatierte Fettsäuremonoglyceride, Sulfobernsteinsäuremonoalkylester
mit 8 - 18 C-Atomen in der Alkylkette oder Dialkylester
mit 6 - 10 C-Atomen in den Alkylgruppen, Alkylpolyglykolethercarboxylate mit 8 - 18 C-Atomen in der Alkylgruppe
und 2 - 6 Polyglykolethergruppen im Molekül, Acylsarkosine, Acyltauride und Acylistethionate mit 8 - 18 C-Atomen in den Acylgruppen.

Neben den anionischen oberflächenaktiven Stoffen können
zwitterionische oder amphotere Waschaktivstoffe in Mengen
bis zu etwa 1/2 des Gehalts an anionischen oberflächenaktiven Stoffen eingesetzt werden. Als zwitterionische
Waschaktivstoffe kommen z.B. Alkylbetaine, Alkylamidopropylbetaine, Alkylimidazoliniumbetaine, Alkylaminocarbonsäuren mit jeweils 8 - 18 C-Atomen in der Alkyl-

...

gruppe in Frage. Beispiele für solche geeigneten zwitterionischen Tenside sind Kokosalkyldimethylaminoessigsäure, Kokosalkylamidopropyl-dimethylaminoessigsäure oder N-Hydroxyethyl-N-kokosalkylamidoethylglycin.

Bevorzugt ist die erfindungsgemäße Verwendung der kationischen Polymeren in einem Shampoo, welches neben  0,5- 5 Gew.-% des kationischen Polymeren 5 - 30 Gew.-% Alkylethersulfattenside und 1 - 5 Gew.-% eines zwitterionischen oder amphoteren Waschaktivstoffes enthält.

Neben den genannten Komponenten können außerdem rückfettende Bestandteile wie z.B. oxethylierte Fettsäurepartialglyceride oder Glycerinpolyglykolether-Fettsäureester, oxethylierte Sorbitanfettsäureester sowie kosmetische Ölkomponenten eingesetzt werden.

Daneben können die haarkosmetischen Mittel auch nichtionogene Waschaktivstoffe und die üblichen, für die jeweilige Anwendung oder Ausführungsform üblichen Zusatz- und Hilfsmittel, Wirkstoffe, Lösungsmittel, Treibgase, Verdickungsmittel, Farbstoffe, Trübungsmittel, Duftstoffe, Konservierungsstoffe, Stabilisierungsmittel, Puffersubstanzen enthalten.

Die folgenden Ausführungsbeispiele sollen den Gegenstand der Erfindung näher erläutern ohne ihn darauf zu beschränken:

- 10 -

B e i s p i e l e

A) Herstellung der kationischen Polymeren

Beispiel 1: Epoxidierung von Polybutadien

In einer Glasapparatur, bestehend aus 4 1 - Dreihalskolben, Rührer und Intensivkühler, wurden 540 g eines Polybutadiens, dessen Viskosität bei 20°C nach DIN 53015 ca. 3 Poise und desser Iodzahl (nach Wijs) ca. 450 g/100 g betrug und welches überwiegend 1.4-cis-Doppelbindungen aufwies (Polyöl hüls[R] 130), in 2000 ml Chloroform gelöst. Zu der auf 50°C erwärmten Lösung wurden unter starkem Rühren in einem Zeitraum von 7,5 Std. 190 g 40 %ige Peressigsäure (in Eisessig), welche 19 g Natriumacetat gelöst enthält, so zugetropft, daß ein stetiger, leichter Rückfluß des durch die Reaktionswärme siedenden Chloroforms stattfand. Das Reaktionsgemisch wurde danach noch 2 Std. bei 50°C gerührt und dann auf 30°C abgekühlt. Das Epoxid wurde durch Eingießen des Ansatzes in 12000 ml Methanol unter stetigem Rühren ausgefällt. Das am Behälterboden abgeschiedene, viskose Öl wurde abgetrennt, noch dreimal mit 5 1 Methanol gemischt und so bis zur Neutralität gewaschen. Das ölige Reaktionsprodukt wurde dann im Vakuum von flüchtigen Anteilen befreit. Die Ausbeute betrug 95 % der Theorie. Das Produkt enthielt 2,5 Gew.-% Epoxid-Sauerstoff.

...

0119454
HENKEL KGaA
ZR-FE/Patente

- 11 -

### Beispiel 2 - 8

Die Epoxidierung wurde ausgehend von 540 g Polybutadien analog Beispiel 1 durchgeführt, wobei die aus Tabelle 1 ersichtlichen Mengen Peressigsäure (40 %ig in Eisessig) eingesetzt und die angegebenen Ausbeuten und Epoxid-Sauerstoffgehalte erhalten wurden.

### T A B E L L E  1

| Beispiel | Menge Peressigsäure (40 %ig in Eisessig) | Ausbeute [% d.Th.] | % Epoxid-Sauerstoff [Gew.-%] |
|----------|------------------------------------------|--------------------|------------------------------|
| 1 | 190 g | 95 | 2,5 |
| 2 | 285 g | 93 | 3,6 |
| 3 | 380 g | 88,5 | 4,7 |
| 4 | 570 g | 83,7 | 6,7 |
| 5 | 665 g | 64,3 | 7,4 |
| 6 | 950 g | 79,0 | 9,3 |
| 7 | 1140 g | 71,1 | 10,3 |
| 8 | 1425 g | 47,5 | 5,8 |

. . .

### Beispiel 9 :  Umsetzung der epoxidierten Polybutadiene mit Dimethylamin

50 g epoxidiertes Polybutadien (Beispiel 1) wurden in 600 g Dioxan gelöst, mit 50 g einer wässrigen, 40 %igen Lösung von Dimethylamin versetzt und in einen Autoklaven aus V4A-Stahl gegeben. Nach zweimaligem Spülen mit Stickstoff wurde bei einem Anfangsdruck von 5 bar gerührt und dabei auf 200°C aufgeheizt, wobei sich ein Maximaldruck von 32 bar einstellte. Nach Abkühlung auf 20°C wurde der Autoklav  entspannt und geöffnet und das Reaktionsprodukt durch Eingießen in 2,5 l destilliertes Wasser ausgefällt. Das isolierte Umsetzungsprodukt wurde durch Auflösen in Dioxan und Ausfällen in Wasser gereinigt und nach dem Trocknen durch die Aminzahl charakterisiert. Das Umsetzungsprodukt wies eine Aminzahl von 55,3 auf. Die Ausbeute betrug 60 % der Theorie.

### Beispiel 10 - 16

Die Umsetzung der epoxidierten Polybutadiene (Beispiel 2 - 8) wurde ausgehend von 50 g der Epoxide in 600 ml Dioxan analog Beispiel 9 durchgeführt, wobei die aus Tabelle 2 ersichtlichen Amine verwendet wurden und Umsetzungsprodukte mit den aus Tabelle 2 ersichtlichen Aminzahlen erhalten wurden.

...

## T A B E L L E 2

| Beispiel | eingesetztes Epoxid | eingesetzter Amin | | Umsetzungsprodukt |
| | | Art | Menge | Aminzahl |
|---|---|---|---|---|
| 9 | Beispiel 1 | Dimethylamin[+] | 50 g | 55,3 |
| 10 | Beispiel 2 | Dimethylamin[+] | 70 g | 69,1 |
| 11 | Beispiel 5 | Dimethylamin[+] | 90 g | 134,8 |
| 12 | Beispiel 5 | Monoethanolamin | 61 g | 98,5 |
| 13 | Beispiel 6 | Dimethylamin[+] | 120 g | 107,8 |
| 14 | Beispiel 6 | Methylamin[+] | 225 g | 240,4 |
| 15 | Beispiel 8 | Dimethylamin[+] | 105 g | 168,2 |
| 16 | Beispiel 8 | Morpholin | 81 g | 153,8 |

[+] 40 %ige wässrige Lösung

Patentanmeldung D 6699 EP

HENKEL KGaA
ZR-FE/Patente

## Herstellung der Hydrochloride

Die Amin-Umsetzungsprodukte gemäß Beispiel 9 - 16 wurden in Dioxan gelöst und mit einer aus der Aminzahl errechneten äquivalenten Menge 18 %iger wässriger Salzsäure versetzt. Dann wurde das Lösungsmittel durch Abdunsten im Vakuum entfernt.

## Beispiel 17 : Quaternierung mit Glycidyltrimethylammoniumchlorid

10 g des Umsetzungsproduktes nach Beispiel 9 wurden in 100 ml Dioxan gelöst, mit 50 mg KOH versetzt und mit 1,8 g Glycidyltrimethylammoniumchlorid (80 %ig, feucht) gemischt. Das Reaktionsgemisch wurde innerhalb von 2 Std. zum Sieden unter Rückfluß erhitzt und weitere 5 Std. bei dieser Temperatur gehalten. Dabei trat eine leichte Trübung ein. Dann wurde das Reaktionsgemisch auf ein Volumen von 50 ml eingeengt und durch Eingießen in 250 ml destillierten Wassers ausgefällt. Die ausgefällte Substanz wurde abgetrennt und getrocknet. Die Ausbeute betrug 9,7 g (d.s. 82,2 % d.Th.).

Zur Überführung in das Hydrochlorid wurde analog Beispiel 9 - 16 verfahren.

## Beispiel 18 und 19

Analog Beispiel 17 wurden die in Tabelle 3 angegebenen Amin-Umsetzungsprodukte mit den angegebenen Mengen Glycidyl-trimethylammoniumchlorid (GMAC) alkyliert und die angegebenen Ausbeuten an Hydrochlorid erhalten.

...

# T A B E L L E   3

| Beispiel | Amin-Umsetzungsprodukt | GMAC 80 %ig | Ausbeute % d.Th. | $Cl^{(-)}$ (Gew.-%) |
|---|---|---|---|---|
| 17 | Beispiel  9 | 1,8 g | 82,2 | 0,48 |
| 18 | Beispiel 10 | 2,2 g | 72,9 | 2,18 |
| 19 | Beispiel 11 | 4,4 g | 55,6 | 0,36 |

...

Beispiel 20 : Quaternierung mit Ethylenoxid

20 g des Umsetzungsproduktes nach Beispiel 10 wurden in 400 ml trockenem Dioxan gelöst und mit 0,5 g Natriummethylat versetzt. Diese Lösung wurde in einen Autoklaven überführt, mit 5,4 g Ethylenoxid (d.s. ca. 5 Mol/Mol Amingruppe) versetzt, auf 70°C erwärmt und 5 Std. bei dieser Temperatur gerührt. Der Druck, welcher zu Beginn 5 bar beträgt, sank in dieser Zeit auf 4,1 bar ab. Nach dem Abkühlen wurde das Reaktionsgemisch unter vermindertem Druck zur Trockene eingeengt. Aus der erhaltenen Ausbeute von 21,1 g errechnet sich eine Anlagerung von ca. 1 Mol Ethylenoxid pro Aminogruppe.

Durch Auflösen in Dioxan, Zugabe von ca. 5 g 18 %iger Salzsäure und Einengen zur Trockene wurde das Quaternierungsprodukt in das gut wasserlösliche Hydrochlorid überführt.

B) Anwendungsbeispiele

Beispiel 21 : Shampoo

| | | |
|---|---|---|
| Fettalkohol ($C_{12-18}$) + 2EO-sulfat, Na-Salz (28 %ig) | 30 | Gew.-% |
| Kokosalkylamidopropyl-betain (30 %ig) | 15 | Gew.-% |
| kationisches Polymer Beispiel 10 (Hydrochlorid) | 2,0 | Gew.-% |
| Formaldehyd (40 %ig) | 0,2 | Gew.-% |
| Wasser, Parfümöl, Farbstoffe | ad 100 | Gew.-% |

...

- 17 -

### Beispiel 22 : Haarspülung

| | | |
|---|---|---|
| Cetylalkohol (Lorol$^{(R)}$ C16) | 1,5 | Gew.-% |
| Glycerin-mono/distearat (Cutina$^{(R)}$ GMS) | 1,5 | Gew.-% |
| kationisches Polymer Beispiel 16 (Hydrochlorid) | 1,5 | Gew.-% |
| Cetyltrimethylammoniumchlorid (25 %ig) | 2,0 | Gew.-% |
| Wasser, Parfümöl, Farbstoff | ad 100 | Gew.-% |

### Beispiel 23 : Haarfärbemittel (braun)

| | | |
|---|---|---|
| Talgfettalkohol | 8,0 | Gew.-% |
| Fettalkohol ($C_{12-18}$) + 2EO-sulfat Na-Salz (28 %ig) | 25,0 | Gew.-% |
| kationisches Polymer Beispiel 18 | 3,0 | Gew.-% |
| p-Toluylendiamin | 1,5 | Gew.-% |
| 2,4-Diaminoanisol | 0,04 | Gew.-% |
| p-Aminophenol | 0,23 | Gew.-% |
| Resorcin | 0,4 | Gew.-% |
| Ammoniak, 25 %ig | 4,5 | Gew.-% |
| Wasser | ad 100 | Gew.-% |

. . .

Patentansprüche

1. Verwendung von kationischen Polymeren, wie sie erhältlich sind durch

- Epoxidation von 1.3-Dien-Homo- oder Copolymerisaten mit mindestens 10 1,3-Dien-Einheiten bis zu einem Umsatz von mindestens 10 % der vorhandenen Doppelbindungen,

- Umsetzung der epoxidierten Poly-1,3-diene mit primären, sekundären oder tertiären Aminen der allgemeinen Formel $R^1NR^2R^3$, in der $R^1$, $R^2$ und $R^3$ Wasserstoff, eine Niedrigalkylgruppe mit 1 - 4 C-Atomen, eine Hydroxyalkylgruppe mit 2 - 4 C-Atomen oder $R^2$ und $R^3$ gemeinsam mit dem Stickstoffatom einen Morpholin-, Piperidin- oder Piperazinring bilden und wenigstens eine Gruppe $R^1$, $R^2$ oder $R^3$ ungleich Wasserstoff ist,

- Überführung der Umsetzungsprodukte mit Mineralsäuren oder niedermolekularen Carbonsäuren in die Salzform, bevorzugt in die Hydrochloride, oder Überführung der polymeren tertiären Amine in quartäre Ammoniumsalze durch Alkylierung mit Verbindungen aus der Gruppe 3-Chlor-2-hydroxypropyltrimethylammoniumchlorid, RCl, RBr, $R_2SO_4$, worin R eine Methyl- oder Ethylgruppe darstellt, oder durch Anlagerung von Ethylenoxid, Propylenoxid, Glycid oder Glycidyltrimethylammoniumchlorid und Überführung in die Salzform, bevorzugt in die Hydrochloride, als antistatisch wirksame Zusätze in haarkosmetischen Mitteln.

...

2. Verwendung nach Anspruch 1, dadurch gekennzeichnet, daß flüssige Butadien-homo- oder -copolymerisate mit mindestens 10 Butadien-Einheiten eingesetzt werden.

3. Verwendung nach Anspruch 2, dadurch gekennzeichnet, daß flüssige Butadienhomopolymerisate eingesetzt werden, die an den Doppelbindungen zu über 50 % 1,4-Cis-Konfiguration aufweisen.

4. Verwendung nach Anspruch 1 bis 3, dadurch gekennzeichnet, daß die epoxidierten Poly-1,3-diene mit Dimethylamin oder Morpholin umgesetzt und die Umsetzungsprodukte in die Hydrochloride überführt sind.

5. Verwendung nach Anspruch 1 bis 3, dadurch gekennzeichnet, daß die epoxidierten Poly-1,3-diene mit Dimethylamin umgesetzt und die Umsetzungsprodukte durch Alkylierung mit Glycidyltrimethylammoniumchlorid oder 3-Chlor-2-hydroxypropyltrimethylammoniumchlorid in die quartären Ammoniumsalze überführt sind.

6. Verwendung nach Anspruch 1 bis 3, dadurch gekennzeichnet, daß die epoxidierten Poly-1,3-diene mit Dimethylamin umgesetzt und die Umsetzungsprodukte durch Anlagerung von Ethylenoxid quaterniert und in die Salzform überführt sind.

7. Verwendung nach Anspruch 1 - 6, dadurch gekennzeichnet, daß die kationischen Polymeren in einer Menge von 0,1 bis 10 Gew.-%, bevorzugt 0,5 - 5,0 Gew.-%, eingesetzt werden.

. . .

8. Verwendung nach Anspruch 1 - 7, dadurch gekennzeichnet, daß neben den kationischen Polymeren anionische oberflächenaktive Stoffe und übliche Hilfs- und Zusatzmittel eingesetzt werden.

9. Verwendung nach Anspruch 7 und 8 in einem Shampoo, dadurch gekennzeichnet, daß neben 0,5 - 5,0 Gew.-% des kationischen Polymeren 5 - 30 Gew.-% Alkylethersulfattenside und 1 - 5 Gew.-% eines zwitterionischen oder amphoteren Waschaktivstoffes eingesetzt werden.